# EUROPÄISCHE PATENTANMELDUNG

(11) **EP 2 388 034 A1**
(43) Veröffentlichungstag der Anmeldung: **23.11.2011**
(21) Anmeldenummer: 11162532.3
(22) Anmeldetag: 15.04.2011
(51) Int. Cl.: A61M 11/04, A61M 35/00, A61H 33/06

(54) **Verfahren zum Behandeln eines menschlichen Körperteils mit Dampf sowie Vaporisator zum Applizieren von Dampf an ein menschliches Körperteil**

(30) Priorität: 04.05.2010 DE 102010016783
(71) Anmelder: WIK Far East Ltd., North Point, Hong Kong (CN)
(72) Erfinder: Kock, Marwin, 45239 Essen (DE); Hafemann, Klaus, 47445 Moers (DE)
(74) Vertreter: Haverkamp, Jens

(57) **Zusammenfassung**

Ein Vaporisator (1) zum Applizieren von Dampf an ein menschliches Körperteil, umfasst:
- ein Vorratsbehältnis (2) zum Bevorraten einer Behandlungsflüssigkeit, insbesondere Wasser,
- eine Pumpe (4), angeschlossen an das Vorratsbehältnis zum Fördern von Behandlungsflüssigkeit,
- einen Verdampfer (5) zum Verdampfen von geförderter Behandlungsflüssigkeit,
- ein dem Verdampfer nachgeschaltetes Mischraum (8),
- eine Einrichtung (10) zum Zuführen eines zum Kühlen des Dampfstroms dienenden Gasstromes, insbesondere Umgebungsluft, und
- ein Behandlungsgefäss (13), in das der Niedrigtemperaturdampf eingeleitet wird. Es wird auch ein entsprechendes Verfahren zum Behandeln eines menschlichen Körperteils mit Dampf beschrieben. Das Verfahren umfasst folgende kontinuierlich durchgeführten Schritte:
- Verdampfen von Behandlungsflüssigkeit unter Ausbildung eines Dampfstromes,
- Abkühlen des Dampfstromes durch Zuführen eines gegenüber der Temperatur des Dampfes kühleren Gases in den Dampfstrom, und
- Einleiten des durch die Abkühlung bereitgestellten Niedrigtemperaturdampfstromes in ein Behandlungsgefäss, ausgebildet zum Einsetzen oder Anlegen eines mit dem Niedrigtemperaturdampf zu behandelnden Körperteils.

## Beschreibung

Die Erfindung betrifft ein Verfahren zum Behandeln eines menschlichen Körperteils mit aus einer Behandlungsflüssigkeit, insbesondere Wasser, erzeugtem Dampf. Ferner betrifft die Erfindung einen Vaporisator zum Applizieren von Dampf an ein menschliches Körperteil.

Für die äußerliche Behandlung von menschlichen Körperteilen, beispielsweise der Füße, der Hände oder des Gesichtes einer Person, werden Bäder verwendet, bei Füßen Fußbäder. Derartige Fußbäder verfügen über eine mit der Behandlungsflüssigkeit befüllbare Wanne und hinreichend Raum, dass die Füße einer Person darin einsetzbar sind. Typischerweise umfasst ein solches Fußband eine Heizeinrichtung zum Erwärmen des Wassers. Zumeist verfügen derartige Fußbäder zudem über Einrichtungen, mit denen ein Fuß massiert werden kann. Je nach der gewünschten Massagetätigkeit, befinden sich diese Einrichtungen innerhalb der Wanne oder in einem die beiden Fußbereiche der Wanne trennende Mittelsteg oberhalb des Flüssigkeitsspiegels. Bekannt ist ferner, in das Bad Luft-oder Wasserströme einzudüsen, um den Effekt eines Whirlpools zu erreichen.

Auch wenn sich mit einem solchermaßen konzipierten Behandlungsgerät unterschiedliche Anwendungen und Behandlungen realisieren lassen, wäre es wünschenswert, wenn ebenfalls Behandlungsgeräte bekannt wären, mit denen menschliche Körperteile, beispielsweise die Füße, mit feuchtem Dampf behandelt werden könnten. Hierbei ist zu berücksichtigen, dass eine solche Behandlung naturgemäß nicht mit Heißdampf erfolgen kann, was zu Verbrühungen führen würde.

Ausgehend von diesem Bedürfnis liegt der Erfindung daher die Aufgabe zugrunde, ein Verfahren sowie einen Vaporisator bereitzustellen, mit dem mit technisch einfachen Mitteln Niedrigtemperaturdampf mit hinreichendem Feuchtigkeitsgehalt bereitgestellt werden kann.

Gelöst wird diese Aufgabe durch ein Verfahren, welches durch folgende Schritte gekennzeichnet ist:
- Verdampfen von Behandlungsflüssigkeit unter Ausbildung eines Dampfstromes,
- Abkühlen des Dampfstromes durch Zuführen eines gegenüber der Temperatur des Dampfes kühleren, insbesondere auf Umgebungstemperatur befindlichen Gases, insbesondere Umgebungsluft in den Dampfstrom, und
- Einleiten des durch die Abkühlung bereitgestellten Niedrigtemperaturdampfstromes in ein Behandlungsgefäß, ausgebildet zum Einsetzen oder Anlegen eines mit dem Niedrigtemperaturdampf zu behandelnden Körperteils.

Gelöst wird diese Aufgabe ferner durch einen Vaporisator zum Applizieren von Dampf an ein menschliches Körperteil, welcher Vaporisator folgende Elemente umfasst:
- ein Vorratsbehältnis zum Bevorraten einer Behandlungsflüssigkeit, insbesondere Wasser,
- einer Pumpe, angeschlossen an das Vorratsbehältnis zum Fördern von Behandlungsflüssigkeit,
- einen Verdampfer zum Verdampfen von von der Pumpe geförderter Behandlungsflüssigkeit,
- einen dem Verdampfer nachgeschalteten Mischraum,
- eine Einrichtung zum Zuführen eines zum Kühlen des Dampfstroms dienenden Gasstromes, insbesondere auf Umgebungstemperatur befindliche Umgebungsluft und
- ein Behandlungsgefäß, in das der Niedrigtemperaturdampf eingeleitet wird.

Bei diesem Verfahren - gleiches gilt entsprechend für den vorbeschriebenen Vaporisator - wird zunächst an einem Verdampfer Behandlungsflüssigkeit verdampft. Als Behandlungsflüssigkeit wird typischerweise Wasser eingesetzt werden. Je nach gewünschter Behandlung können auch andere Flüssigkeiten, vorzugsweise wässrige Flüssigkeiten - verdampft werden. Zu diesem Zweck wird aus einem Vorratsbehältnis Behandlungsflüssigkeit mittels einer Pumpe abgezogen und kontinuierlich oder pulsierend einem Verdampfer zugeführt. Auf diese Weise wird ein Heißdampfstrom erzeugt. Zum Kühlen dieses feuchten Heißdampfstromes wird in diesen ein kühlendes Gas, insbesondere auf Umgebungstemperatur befindliche Umgebungsluft zugeführt. Das zugeführte und bezüglich der Temperatur des Heißdampfstromes kühlere Gas durch mischt sich mit dem Heißdampfstrom und kühlt diesen auf eine für die Behandlung vorgesehene Temperatur. In Abhängigkeit von der zugeführten Luftmenge bzw. von dem Mischungsverhältnis zwischen Heißdampfstrom und zugeführter Kühlluft ist neben typischerweise auch anderen Möglichkeit die Temperatur des Niedrigtemperaturdampfes für die Behandlung einstellbar. Der auf diese Weise gekühlte Heißdampfstrom ist im Rahmen dieser Ausführungen als Niedrigtemperaturdampf angesprochen. Von Besonderheit ist, dass der zugeführte Luftstrom einerseits zwar den Heißdampf abkühlt, jedoch gleichzeitig durch die zugeführte Luftmenge Sorge dafür trägt, dass im Zuge des Mischens Dampfanteile nicht oder im Wesentlichen nicht kondensieren. Die zugeführte kühlende Luft ist zu diesem Zweck hinsichtlich ihrer Wasseraufnahmekapazität nicht gesättigt. Dieses dürfte regelmäßig bei Zuführen von auf Umgebungstemperatur befindlicher Luft der Fall sein. Auf diese Weise ist mit einfachen Mitteln ein Niedrigtemperaturdampf mit einem hohen Feuchtigkeitsgehalt erzeugbar, so dass dieser ohne weiteres zur Behandlung eines menschlichen Körperteils appliziert werden kann. Für die Zwecke des Förderns des kühlenden Gasstromes, etwa des zugeführten Umgebungsluftstromes dient typischerweise ein Gebläse mit einem Ventilator. Bevorzugterweise sind die Aktoren eines solchermaßen konzipierten Vaporisators - die Pumpe, der Verdampfer sowie das Gebläse - hinsichtlich ihrer Leistung und/oder auch ihrer Pumpenfrequenz regulierbar. Zu diesem Zwecke sind die Aktoren an eine Steuereinheit angeschlossen. In Abhängigkeit von der jeweilig benutzerseitig vorgenommenen Einstellung des gewünschten Dampfes, wie Temperatur, Wassergehalt und Menge, erfolgt dann eine entsprechende Ansteuerung der an der Erzeugung des Niedrigtemperaturdampfes beteiligten Aktoren.

Der Verdampfer kann beispielsweise mittels eines PTC-Heizelementes betrieben werden. Ausgenutzt wird bei diesem Ausführungsbeispiel die Leistungscharakteristik eines solchen Heizelementes, da dieses hinsichtlich seiner Zieltemperatur eingestellt bzw. in Bezug auf eine bestimmte Zieltemperatur ausgeführt sein kann. Zudem ist die Heizkurve eines solchen PTC-Heizelementes von seiner aktuellen Temperatur abhängig. Daher kann ein solches Heizelement länger in einem Temperaturbereich gehalten werden, in dem ein Dampf mit besonders hoher Feuchtigkeitstragung erzeugt werden kann. Die Temperatur für einen solchen Dampf liegt bei etwa 135 Grad Celsius. Aufgrund der Trägheit eines PTC-Heizelementes kann die Zufuhr von zu verdampfendem Wasser in Abhängigkeit von der aktuellen Temperatur des PTC-Heizelementes gesteuert werden. Bei einer solchen Ausgestaltung wird zu verdunstendes Wasser pulsierend dem Verdampfer zugeführt. Bei dieser Betriebsweise kann der Verdampfer zwischen einer Temperatur von 130 Grad Celsius und 140 Grad Celsius durch entsprechende Wasserzuführung gehalten werden. Insofern ist der Einsatz eines oder auch mehrerer PTC-Heizelemente zum Erzeugen des gewünschten Dampfes zweckmäßig.

In einem bevorzugten Ausführungsbeispiel wird der kühlende Gasstrom in einer Mischkammer dem Heißdampfstrom beigemengt. Die Mischkammer zeichnet sich durch einen verglichen mit der Dampfzuführung größeren Querschnitt aus. Die Mischkammer kann Dampf bzw. Kühlgas lenkende Einbauten enthalten, um eine Durchmischung des Heißdampfes mit dem kühlenden Gas zu unterstützen. In einer solchen Mischkammer oder auch in Strömungsrichtung des Dampfes vor dieser kann - wenn gewünscht - zudem ein Verdunster angeordnet oder auch einsetzbar sein. Über einen solchen Verdunster, der gemäß einem Ausführungsbeispiel als Schale oder poröser Kunststoff- oder Metallbehälter, beispielsweise aus einem Sinterwerkstoff hergestellt, konzipiert sein kann, können Zusatzstoffe oder Zusatzstoffgemische, wie beispielsweise ätherische Öle oder andere Duftstoffe oder dem zu applizierenden Dampf beizumengende Behandlungsstoffe enthalten sein. Vorzugsweise befindet sich ein solcher Verdunster in einer Anordnung innerhalb der Mischkammer oder unmittelbar vor der Mischkammer, dass diese von dem Heißdampfstrom, dem kühlenden Gasstrom oder einem Mischgasstrom angeströmt ist.

Zusätzlich kann ein solcher Vaporisator mit einer in den Behandlungs- bzw. Nutzraum mündende Sprüheinrichtung ausgerüstet sein. Typischerweise wird man die Sprühvorrichtung, wenn eingebaut, zum Zuführen von Kaltwasser nutzen, beispielsweise um eine thermische Wechselbehandlung unterstützen zu können.

In einer anderen Ausgestaltung ist vorgesehen, dass in den Nutzraum zusätzlich Umgebungsluft eingeblasen werden kann. Dieses kann mit ein und demselben Gebläse erzielt werden, wenn in den ausgangsseitigen Strömungskanal des Gebläses ein Stellglied zum Leiten des geförderten Luftstromes entweder auf direktem Wege in den Nutzraum und/oder in die Mischkammer angeordnet wird. Das wechselweise Beaufschlagen des Nutzraumes mit feuchtigkeitstragendem Niedertemperaturdampf und zugeführter Umgebungsluft unterstützt den Wechselbadeffekt, da die zugeführte Luft den Dampf verdrängt und den Verdunstungskälteeffekt verstärkt. Zusätzlich und auch unterstützend bei einer solchen Behandlung kann die vorbeschriebene Sprüheinrichtung genutzt werden.

Weitere Vorteile und Ausgestaltungen der Erfindung ergeben sich aus der nachfolgenden Beschreibung von Ausführungsbeispielen unter Bezugnahme auf die beigefügten Figuren. Es zeigen:
- **Fig. 1**:: eine schematisierte Darstellung eines Vaporisators gemäß einem ersten Ausführungsbeispiel,
- **Fig. 2**:: eine schematisierte Darstellung eines Vaporisators gemäß einem weiteren Ausführungsbeispiel und
- **Fig. 3**:: eine schematisierte Schnittdarstellung eines Verdampfers.

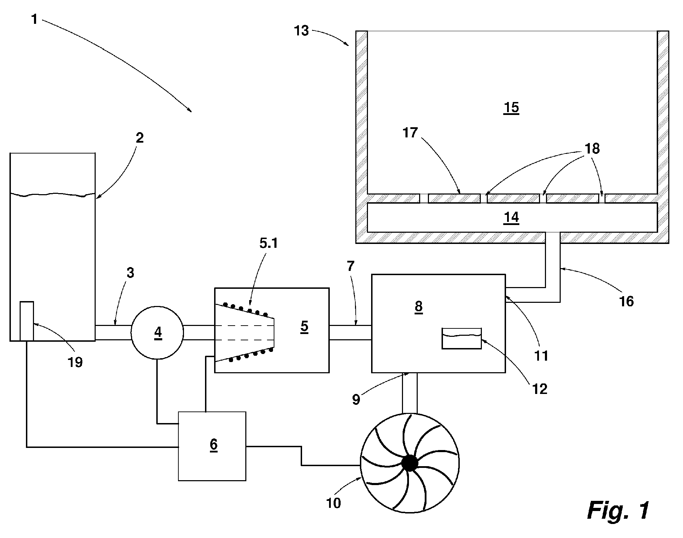

Figur 1 zeigt einen Vaporisator 1 zum Applizieren eines Niedrigtemperaturdampfes an ein menschliches Körperteil in einer schematisierten Darstellung. In natura ist der Vaporisator 1 bei diesem Ausführungsbeispiel in ein herkömmliches Fußbad integriert. Somit verfügt das Fußbad neben den ansonsten üblichen Aggregaten zudem über die dem Vaporisator 1 zugehörigen Elemente.

Der Vaporisator 1 umfasst ein Vorratsbehältnis 2 zum Bevorraten von Wasser, welches bei diesem Ausführungsbeispiel als Behandlungsflüssigkeit verwendet wird. Das Vorratsbehältnis 2 ist ein innerhalb des Fußbades befindlicher Tank. Der Ausgang des Vorratsbehältnisses 2 ist an eine Förderleitung 3 angeschlossen, über die Wasser aus dem Vorratsbehältnis 2 durch eine bei dem dargestellten Ausführungsbeispiel elektromotorisch betriebene Pumpe 4 einem in einer Verdampferkammer 5 angeordneten Verdampfer 5.1 zugeführt wird. Die Pumpe 4 und der Verdampfer 5 sind hinsichtlich ihrer Leistung regulierbar und zu diesem Zwecke an eine Steuereinheit 6 angeschlossen. Mithin wird über die Steuereinheit 6 die Leistungsaufnahme der Pumpe 4 sowie des Verdampfers 5 angesteuert. Damit kann die Pumpe auch pulsierend angesteuert werden. In nicht näher dargestellter Art und Weise ist an die Steuereinheit 6 ebenfalls ein Stellglied angeschlossen, mit dem ein Benutzer den Vaporisator ein- und ausschalten kann und mit dem ein Benutzer bestimmte Dampfparameter, beispielsweise Temperatur und Feuchtigkeitsgehalt, einstellen kann.

Der Ausgang des Verdampfers 5 ist über eine Förderleitung 7 an eine Mischkammer 8 angeschlossen. Somit wird der durch den Verdampfer 5 bereitgestellte Heißdampf als Heißdampfstrom über die Förderleitung 7 in die Mischkammer 8 gefördert. In die Mischkammer 8 mündet des Weiteren der Ausgang 9 eines Gebläses 10, welches bei dem dargestellten Ausführungsbeispiel als Förderglied einen Ventilator umfasst. Über das Gebläse 10 wird auf Umgebungstemperatur befindliche Umgebungsluft in die Mischkammer 8 eingebracht. Die hinsichtlich ihrer Temperatur in die Mischkammer 8 zugeführte Umgebungsluft vermischt sich mit dem Heißdampfstrom und kühlt diesen in Abhängigkeit von der Temperaturdifferenz zwischen dem Heißdampfstrom und dem zugeführten Umgebungsluftstrom und dem Mischungsverhältnis, mit dem diese beiden Ströme zugeführt werden, ab. Das Mischungsverhältnis wird eingestellt, damit am Ausgang 11 der Mischkammer ein Niedrigtemperaturdampfstrom austritt. Die Temperatur entspricht der Behandlungstemperatur. Gleichzeitig wird das Verhältnis der Mischung von Heißdampfstrom und zugeführter Umgebungsluft so eingestellt, dass die durch den Heißdampfstrom in die Mischkammer 8 eingebrachte Feuchtigkeit nicht kondensiert. Mithin bleibt der Feuchtigkeitsgehalt des Heißdampfes zumindest im Wesentlichen erhalten. Daher ist im Gegensatz zur weitestgehenden Sättigung des Heißdampfstromes hinsichtlich seiner Wasseraufnahmekapazität der über das Gebläse 10 zugeführte kühlende Umgebungsluftstrom hinsichtlich seiner Wasseraufnahmekapazität ungesättigt.

Innerhalb der Mischkammer 8 befindet sich ein Verdunster 12. Der Verdunster 12 ist in nicht dargestellter Art und Weise in die Mischkammer 8 durch Öffnen einer Klappe einsetzbar und aus dieser herausnehmbar. Der Verdunster 12 dient zur Aufnahme von Behandlungsstoffen, die mit dem Niedrigtemperaturdampf über den Ausgang 11 dem zu behandelnden Körperteil, in diesem Fall den Füßen, zugeführt werden soll. Bei den Behandlungsstoffen kann es sich beispielsweise um ätherische Öle handeln.

Dem Ausgang 11 der Mischkammer 8 nachgeschaltet ist das eigentliche Behandlungsgefäß 13. Dieses ist in einen Entspannungsraum 14 und einen Nutzraum 15 unterteilt. In dem Entspannungsraum 14 wird der über eine Leitung 16 zugeführte feuchtigkeitsbeladene Niedrigtemperaturdampf über eine gewisse Fläche verteilt. Mithin dient der Entspannungsraum 14 als Ausgangssammler. In die den Entspannungsraum 14 von dem Nutzraum 15 trennende Wand 17, die bei dem dargestellten Ausführungsbeispiel die Bodenwand des Nutzraumes 15 ist, sind mehrere Durchbrechungen 18 eingebracht, durch die der Niedrigtemperaturdampf in den Nutzraum 15 eintritt. Die Durchbrechungen 18 sind über den Boden 17 oder auch in der Seitenwand des Nutzraumes 15 verteilt, damit die in das Fußbad eingesetzten Füße aus unterschiedlichen Richtungen dampfbeaufschlagt sind.

Verfügt das Fußbad neben der vorbeschriebenen Vaporisatoreinrichtung auch über eine Einrichtung zum Zuführen von Luft in den Nutzraum 15, wenn dieser wasserbefüllt ist, kann das Gebläse 10 gleichfalls dem Zweck des Zuführens von Luft in den Nutzraum dienen. Eine solchermaßen vorgesehene Luftzufuhr wird über eine andere Wirksamkeit realisiert. Dann befindet sich in der Verbindung zwischen dem Gebläse 10 und der Mischkammer 8 ein Ventil, welches bei einem solchermaßen vorgesehenen Betrieb geschlossen ist. Das Gebläse fördert dann die in den Nutzraum über Düsen einzubringende Luft über eine weitere Luftzuführleitung (in der Figur nicht dargestellt). Das Gebläse 10 mit seinem Ventilator ist leistungsregulierbar und zu diesem Zweck an die Steuereinheit 6 angeschlossen.

Das Vorratsbehältnis 2 verfügt über einen Füllstandssensor 19, der ebenfalls von der Steuereinheit 6 auslesbar ist. Ist der Füllstand innerhalb des Vorratsbehältnisses 2 zu gering oder fällt dieser unter eine vorbeschriebene Mindestmenge ab, können aus Sicherheitsgründen die Pumpe 4 und der Verdampfer 5 ausgeschaltet werden. Gleichzeitig wird ein Signal generiert, mit dem einem Benutzer signalisiert wird, dass Wasser in das Vorratsbehältnis 2 nachzufüllen ist.

Die Steuereinheit 6 steuert in Abhängigkeit von einem vorgegebenen Steueralgorithmus und einer nutzerseitigen Einstellung den Betrieb des Vaporisators 1 bzw. seiner Aktoren - der Pumpe 4, des Verdampfers 5 und des Gebläses 10. Über die Leistungsregulierung der Pumpe 4 und des Verdampfers 5 ist die generierte Heißdampfmenge und die Temperatur des generierten Heißdampfes einstellbar. Die Leistungsregelbarkeit des Gebläses 10 erlaubt das Mischungsverhältnis zwischen dem über die Förderleitung 7 strömenden Heißdampfstrom und dem über das Gebläse 10 zugeführten kühlenden Umgebungsluftstromes einzustellen. Gleichermaßen kann dieses Mischungsverhältnis auch durch entsprechende Ansteuerung der Pumpe 4 sowie des Verdampfers 5 beeinflusst werden.

Bei einer Benutzung eines solchermaßen konzipierten Fußbades kann die Applikation von Niedrigtemperaturdampf, der sich auf einer für einen Benutzer angenehmen Temperatur befindet und daher als "warm" wahrgenommen wird, mit einer über Düsen in den Nutzraum 15 zugeführten Kaltwasser (nicht dargestellt) abwechseln. Ein solche wiederholte abwechselnde Behandlung der Füße mit Niedrigtemperaturdampf und Kaltwasser entspricht einer so genannten Kneipp'schen Anwendung.

In dem Entspannungsraum kondensierte Flüssigkeit kann über eine nicht dargestellte Abzugseinrichtung entfernt werden.

Das Vorsehen des Gebläses 10 bei dem Vaporisator 1 kann ferner dazu genutzt werden, dieses für die Zwecke der Trocknung der Dampfwegsamkeiten einzusetzen. Somit können durch einfachen Nachlauf des Gebläses 10 nach Abschalten der Pumpe 4 und des Verdampfers 5 in der Mischkammer 8 befindliche Feuchtigkeitsreste durch den durch das Gebläse 10 geförderten Luftstrom aufgenommen und über die Leitung 16, den Entspannungsraum 14 und den Nutzraum 15 entfernt werden. Im Zuge eines solchermaßen geförderten Luftstromes wird gleichzeitig die Leitung 16, der Entspannungsraum 14 und der Nutzraum 15 getrocknet. Somit kann durch Betrieb des Gebläses 10 das Gerät nach einem vorzugsweise vorherigem Auswischen desselben vollständig getrocknet werden.

Ist eine Desinfektion gewünscht, kann diesem Luftstrom über eine typischerweise in die Mischkammer 8 mündende Einspritzvorrichtung ein Desinfektionsmittel beigemengt werden.

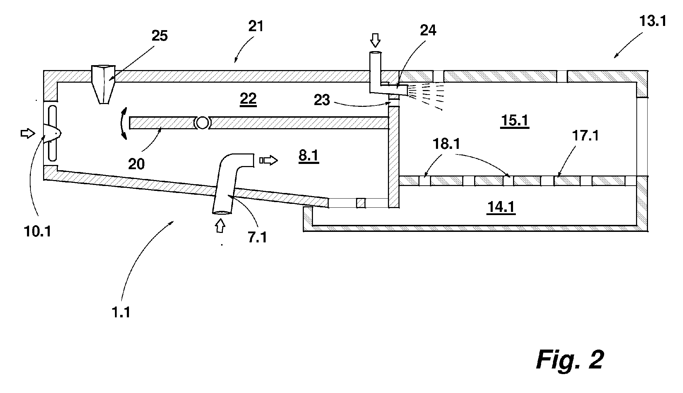

Figur 2 zeigt einen Vaporisator 1.1 gemäß einem weiteren Ausführungsbeispiel. Der Vaporisator 1.1 arbeitet nach demselben Prinzip zum Applizieren eines Niedrigtemperaturdampfes wie dieses zu dem Vaporisator 1 der Figur 1 beschrieben ist. Daher gelten die diesbezüglichen Ausführungen auch für den Vaporisator 1.1.

Der Vaporisator 1.1 verfügt ebenfalls über ein Behandlungsgefäß 13.1. Unterhalb des auch als Behandlungsraum angesprochenen Nutzraumes 15.1 des Behandlungsgefäßes 13.1 befindet sich ein Entspannungsraum 14.1, der ebenso wie bei dem Ausführungsbeispiel der Figur 1 zur Dampfverteilung dient. Der Nutzraum 15.1 ist von dem Entspannungsraum 14.1 durch eine Durchbrechungen 18.1 aufweisende Wand 17.1 getrennt. In den Entspannungsraum 14.1 mündet der Ausgang einer Mischkammer 8.1. Die Mischkammer 8.1 des Vaporisators 1.1 vergrößert sich in Richtung zu dem Ausgang zum Entspannungsraum 14.1 hin. In die Mischkammer 8.1 mündet eine Förderleitung 7.1, durch die von einem Verdampfer erzeugter Heißdampf, wie durch die Pfeile schematisiert dargestellt, zugeführt wird. Die Förderleitung 7.1 ist innerhalb der Mischkammer 8.1 in Strömungsrichtung zum Entspannungsraum 14.1 hin abgewinkelt. Die Mischkammer 8.1 ist an ihrem dem Ausgang zum Entspannungsraum 14.1 hin gegenüber liegenden Ende durch einen nicht dargestellten Aktor ansteuerbare Stellklappe 20 begrenzt. Das Gebläse 10.1 bei dem Vaporisator 1.1 sitzt an einer Stirnseite des sich an den Nutzraum 15.1 anschließenden Gehäuseteils 21. Selbstverständlich kann das Gebläse 10.1 auch außerhalb sitzen; dann mündet die Luftförderleitung vorzugsweise an der in Figur 2 gezeigten Stelle in das Gehäuseinnere. Oberhalb der Mischkammer 8.1 befindet sich ein Luftkanal 22. Der Luftkanal 22 kann ebenso wie die Mischkammer 8.1 durch die Stellklappe 20 verschlossen werden. Somit kann bei einem Betrieb des Gebläses 10.1 in Abhängigkeit von der Stellung der Stellklappe 20 die geförderte Luft entweder in die Mischkammer 8.1 oder in den Luftkanal 22 eingebracht werden. Es versteht sich, dass auch ein Mischbetrieb, bei dem Luft sowohl der Mischkammer 8.1 als auch dem Luftkanal 22 zugeführt wird, denkbar ist. Der Luftkanal 22 mündet bei dem dargestellten Ausführungsbeispiel in den oberen Bereich des Nutzraumes 15.1.

Oberhalb der Mündung 23 des Luftkanals 22 in den Nutzraum 15.1 ist der Ausgang einer Kaltwassersprüheinrichtung 24 angeordnet. Über diese kann Kaltwasser in den Nutzraum 15.1 eingesprüht werden.

In Strömungsrichtung der durch das Gebläse 10.1 geförderten Luft der Stellklappe 20 vorgeschaltet ist in die obere Wand des Gehäuseteils 21 eine Kartusche 25, bei dem dargestellten Ausführungsbeispiel aus Sintermetall, in das Gehäuseteilinnere eingreifend angeordnet. Die Kartusche 25 dient zur Aufnahme von Duft- und/oder Pflegestoffen, die bei einem Betrieb des Vaporisators 1.1 an die durch das Gebläse 10.1 geförderte Luft abgegeben und sodann in den Nutzraum 15.1 transportiert werden.

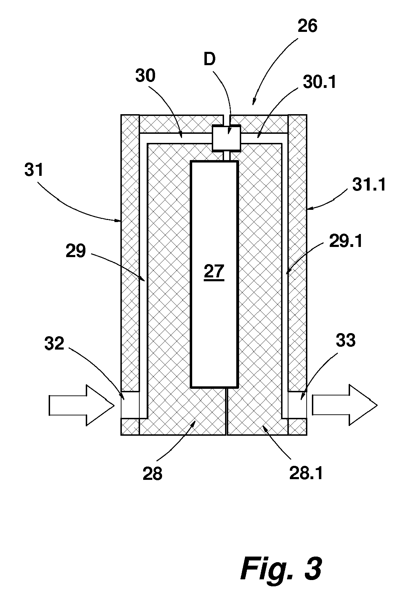

Figur 3 zeigt einen Verdampfer 26, dessen Kern ein PTC-Heizelement 27 ist. Das PTC-Heizelement 27 ist eingebettet in ein Strömungswegsamkeiten enthaltenden Aluminiumblock. Der Aluminiumblock des dargestellten Ausführungsbeispiels ist aus mehreren Teilen zusammengesetzt. Unmittelbar das PTC-Heizelement 27 einfassend sind zwei Blockteile 28, 28.1 vorgesehen. Zwischen diesen ist das PTC-Heizelement 27 unter einer gewissen Vorspannung stehend mit seinen Flachseiten gehalten. Dieses dient zum Bereitstellen eines besonders guten Wärmeüberganges von dem PTC-Heizelement 27 auf die Blockteile 28, 28.1. Das Blockteil 28 verfügt außenseitig über eine sich mäandrierend über die Längserstreckung des Blockteils 28 erstreckenden Kanal 29, der an seinem in Strömungsrichtung befindlichen Ende in einen Verbindungskanal 30 übergeht. Das Blockteil 28.1 ist prinzipiell gleich aufgebaut, jedoch strömt in diesem das Fluid von dem Verbindungskanal 30.1 in den in gleicher Weise mäandrierend konzipierten Strömungskanal 29.1. Die beiden Verbindungskanäle 30, 30.1 sind durch eine Dichtungshülse D miteinander verbunden. Die Dichtungshülse D ist eingesetzt in eine entsprechende Hülsenausnehmung in jeweils einem Blockteil 28 bzw. 28.1. Die Strömungskanäle 29, 29.1 sind durch Fräsen in die jeweiligen Blockteile 28, 28.1 eingebracht.

Verschlossen sind diese Kanäle 29, 29.1 jeweils durch einen Deckel 31, 31.1. In dem Deckel 31 befindet sich eine Zuströmöffnung 32. In dem Deckel 31.1 ein Ausgang 33. Durch den Zustromeingang 32 wird Wasser an den Verdampfer 26 geführt. Aus dem Ausgang 33 tritt Dampf aus, wie dieses schematisiert durch die Blockpfeile in Figur 3 dargestellt ist.

Bei einem Betrieb des Verdampfers 26 wird durch den Eingang 32 Wasser in den Verdampfer 26 eingebracht, verdampft an dem auf entsprechender Temperatur befindlichen Aluminium, tritt durch den Ausgang 33 aus und wird sodann beispielsweise über die Förderleitung 7.1 der Mischkammer 8.1 zugeführt. Im Zuge des Verdampfens wird den Aluminiumblockteilen 28, 28.1 Wärme entzogen. Gleiches gilt sodann für das PTC-Heizelement 27, welches bei entsprechender Wasserbeaufschlagung der Strömungswegsamkeit ebenfalls abgekühlt wird. Durch die Trägheit des PTC-Heizelementes 27 benötigt dieses somit gewisse Ruhezeiten, in denen durch die Aluminiumblockteile 28, 28.1 kein Wasser für die Dampferzeugung hindurchgepumpt wird, damit das PTC-Heizelement 27 wieder auf die vorgesehene Temperatur erwärmt ist. Daher wird man den Verdampfer 26 typischerweise mit einer gepulsten Wasserzuführung steuern, und zwar abhängig von der Temperatur des PTC-Heizelementes.

Für die Zwecke des Bereitstellens einer gleichmäßigen Dampfströmung ist der Querschnitt der Fluidkanäle 29.1 geringfügig kleiner bemessen als derjenige der Strömungskanäle 29. Dadurch wird ein gewisser Staudruck erzeugt mit der Folge, dass der erzeuge Dampf aus dem Ausgang 33 gleichmäßig ausströmt. Bei einem gepulsten Betrieb des Verdampfers 26 trifft das gleichmäßige Ausströmen des Dampfes für die wasserbeaufschlagten Pulszeiten zu.

Die elektrischen/elektronischen Aktoren des Vaporisators 1.1 sind in nicht näher dargestellter Art und Weise an einer Steuereinrichtung angeschlossen und können mithin von dieser angesteuert werden. Aufgrund der unterschiedlichen Möglichkeiten einer Behandlung mit dem Vaporisator 1.1 können von einem Nutzer vorbestimmte Programme ausgewählt werden, die sodann von der Steuereinheit abgearbeitet werden. Hierbei kann es sich um eine reine Dampfbehandlung handeln. Dann befindet sich die Stellklappe 20 in ihrer den Luftkanal 22 verschließenden Stellung. Ebenfalls ist eine Behandlung ohne Dampf möglich. Dann befindet sich die Stellklappe 20 in ihrer den Mischraum 8.1 verschließenden Stellung. Auch ist ein Wechselprogramm möglich, bei dem für eine vorbestimmte Zeit der durch das Gebläse 10.1 generierte Luftstrom durch die Mischkammer 8.1 und entsprechender Dampfzuführung geleitet wird und wechselweise durch den Luftkanal 22. In gleicher Weise kann die Kaltwassersprüheinrichtung ebenfalls Teil eines Programms sein. Auch eine manuelle Steuerung der Kaltwassersprüheinrichtung 24 ist möglich.

Aufgrund der besonders hohen Feuchtigkeitstragung des Dampfes in Form vieler kleiner Wassertröpfchen eignen sich die vorbeschrieben Vaporisatoren bzw. die Behandlungsgefäße besonders, um den darin befindlichen Dampf zu illuminieren, beispielsweise mit einer LED-Beleuchtungseinrichtung. Eine solche Illumination kann farbig auch in Abhängigkeit von dem jeweiligen Behandlungsprogramm oder dem aktuell durchgeführten Behandlungsschritt unterschiedlich farbig ausgeführt sein.

In einer Weiterbildung ist vorgesehen, dem Behandlungsgefäß einen oder mehrere Aufsätze, typischerweise als Deckelaufsätze konzipiert, zuzuordnen, um eine Behandlung unterschiedlicher Körperteile in Abhängigkeit von dem jeweils gewählten Aufsatz zu ermöglichen. So kann beispielsweise ein Gesichtsaufsatz vorgesehen sein, dessen obere Kante an die Kontur des Gesichtes einer Person angepasst ist. Zusätzlich kann bei einer solchen Behandlung der Vaporisator auf ein Podest aufgestellt sein, mit dem dieser für eine ergonomisch günstigere Behandlung geneigt ist.

Die Beschreibung der Erfindung verdeutlicht, dass mit dem beschriebenen Konzept des Vaporisators und dem beschriebenen Verfahren sich ein solcher Vaporisator auch mit geringem Bauvolumen realisieren lässt. Aus diesem Grunde eignet sich diese Konzeption vor allem auch dann, wenn eine Vaporisatorfunktion in ein bestehendes Gerät, wie beispielsweise ein Fußbad oder dergleichen zusätzlich integriert werden soll.

### Bezugszeichenliste

- 1, 1.1: Vaporisator
- 2: Vorratsbehältnis
- 3: Förderleitung
- 4: Pumpe
- 5: Verdampferkammer
- 5.1: Verdampfer
- 6: Steuereinheit
- 7, 7.1: Förderleitung
- 8, 8.1: Mischkammer
- 9: Ausgang
- 10, 10.1: Gebläse
- 11: Ausgang
- 12: Verdunster
- 13, 13.1: Behandlungsgefäß
- 14, 14.1: Entspannungsraum
- 15, 15.1: Nutzraum
- 16: Leitung
- 17, 17.1: Wand
- 18, 18.1: Durchbrechung
- 19: Füllstandssensor
- 20: Stellklappe
- 21: Gehäuseteil
- 22: Luftkanal
- 23: Mündung
- 24: Kaltwassersprüheinrichtung
- 25: Kartusche
- 26: Verdampfer
- 27: PTC-Heizelement
- 28, 28.1: Aluminiumblockteil
- 29, 29.1: Fluidkanal
- 30, 30.1: Verbindungskanal
- 31, 31.1: Deckel
- 32: Eingang
- 33: Ausgang
- D: Dichtungshülse

## Patentansprüche

1. Verfahren zum Behandeln eines menschlichen Körperteils mit aus einer Behandlungsflüssigkeit, insbesondere Wasser, erzeugtem Dampf, **gekennzeichnet durch** folgende kontinuierlich durchgeführten Schritte:
- Verdampfen von Behandlungsflüssigkeit unter Ausbildung eines Dampfstromes,
- Abkühlen des Dampfstromes **durch** Zuführen eines gegenüber der Temperatur des Dampfes kühleren, insbesondere auf Umgebungstemperatur befindlichen Gases, insbesondere Umgebungsluft in den Dampfstrom, und
- Einleiten des **durch** die Abkühlung bereitgestellten Niedrigtemperaturdampfstromes in ein Behandlungsgefäß (13, 13.1), ausgebildet zum Einsetzen oder Anlegen eines mit dem Niedrigtemperaturdampf zu behandelnden Körperteils.

2. Verfahren nach Anspruch 1, **dadurch gekennzeichnet, dass** der Schritt des Verdampfens von Behandlungsflüssigkeit umfasst, dass die Behandlungsflüssigkeit aus einem Vorratsgefäß (2) mittels einer leistungsregulierbaren Pumpe (4) abgepumpt und einem leistungsregulierbaren Verdampfer (5, 26) zugeführt wird.

3. Verfahren nach Anspruch 1 oder 2, **dadurch gekennzeichnet, dass** das zum Kühlen des Dampfstromes zugeführte Gas aktiv in den Dampfstrom hinein gefördert wird.

4. Verfahren nach Anspruch 3, **dadurch gekennzeichnet, dass** eine solche Menge an Gas zugeführt wird, die ausreicht, damit die im Heißdampf enthaltene Behandlungsflüssigkeitsmenge trotz Abkühlung nicht oder zumindest im Wesentlichen nicht kondensiert.

5. Verfahren nach einem der Ansprüche 1 bis 4, **dadurch gekennzeichnet, dass** der zu kühlende Dampfstrom und das zugeführte Gas in einem Mischraum (8, 8.1) zusammengeführt werden.

6. Verfahren nach Anspruch 5, **dadurch gekennzeichnet, dass** in dem Mischraum (8, 8.1) dem Dampf-Gas-Gemisch ein Zusatzstoff oder ein Zusatzstoffgemisch beigemengt wird.

7. Verfahren nach Anspruch 6, **dadurch gekennzeichnet, dass** der Zusatzstoff oder das Zusatzstoffgemisch in dem Mischraum verdunstet und auf diese Weise dem Dampf-Gas-Gemisch beigemengt werden.

8. Verfahren nach einem der Ansprüche 1 bis 7, **dadurch gekennzeichnet, dass** der Niedrigtemperaturdampf vor Einleiten in den Behandlungsraum (15, 15.1) des Behandlungsgefäßes (13, 13.1) zu seiner Verteilung entspannt wird.

9. Vaporisator zum Applizieren von Dampf an ein menschliches Körperteil, umfassend:
- ein Vorratsbehältnis (2) zum Bevorraten einer Behandlungsflüssigkeit, insbesondere Wasser,
- einer Pumpe (4), angeschlossen an das Vorratsbehältnis (2) zum Fördern von Behandlungsflüssigkeit,
- einen Verdampfer (5, 26) zum Verdampfen von von der Pumpe (4) geförderter Behandlungsflüssigkeit,
- einem dem Verdampfer (5, 26) nachgeschalteten Mischraum (8, 8.1),
- eine Einrichtung zum Zuführen eines zum Kühlen des Dampfstroms dienenden Gasstromes, insbesondere auf Umgebungstemperatur befindliche Umgebungsluft und
- ein Behandlungsgefäß (13, 13.1), in das der Niedrigtemperaturdampf eingeleitet wird.

10. Vaporisator nach Anspruch 9, **dadurch gekennzeichnet, dass** die Pumpe (4) und/oder der Verdampfer (5, 26) leistungsregulierbar und zum Zwecke ihrer Ansteuerung an eine Steuereinheit (6) angeschlossen sind.

11. Vaporisator nach Anspruch 9 oder 10, **dadurch gekennzeichnet, dass** die Einrichtung zum Zuführen des Kühlgasstromes ein Gebläse (10, 10.1) umfasst.

12. Vaporisator nach Anspruch 11, **dadurch gekennzeichnet, dass** das Gebläse (10, 10.1) leistungsregulierbar ist und zum Zwecke seiner Ansteuerung an eine Steuereinheit (6) angeschlossen ist.

13. Vaporisator nach einem der Ansprüche 9 bis 12, **dadurch gekennzeichnet, dass** in dem Mischraum (8, 8.1) oder diesem vorgeschaltet ein Verdunstungsbehältnis (12, 25) angeordnet oder darin einsetzbar ist.

14. Vaporisator nach einem der Ansprüche 9 bis 13, **dadurch gekennzeichnet, dass** das Behandlungsgefäß (13, 13.1) über einen Entspannungsraum (14, 14.1) verfügt, der von einem Nutzraum (15, 15.1), in den ein Körperteil ein- oder anlegbar ist, durch eine Wand (17, 17.1) abgetrennt ist, welche Wand (17, 17.1) über Durchbrüche (18, 18.1) zum Einleiten des Niedrigtemperaturdampfes in den Nutzraum (15, 15.1) verfügt.

15. Vaporisator nach einem der Ansprüche 9 bis 14, **dadurch gekennzeichnet, dass** das Behandlungsgefäß (13) als Fußbad konzipiert oder in ein solches integriert ist.
